# EUROPEAN PATENT SPECIFICATION

(11) **EP 0 862 453 B1**
(45) Date of publication and mention of the grant of the patent: **05.04.2006**
(21) Application number: 97936493.2
(22) Date of filing: 13.08.1997
(51) Int. Cl.: A61K 38/18, A61K 48/00, C12P 21/00, C12N 5/10, C12N 5/06, A61P 9/00

(54) **BONE MORPHOGENETIC PROTEIN AND FIBROBLAST GROWTH FACTOR COMPOSITIONS AND METHODS FOR THE INDUCTION OF CARDIOGENESIS**
KNOCHEN MORPHOGENETISCHES PROTEIN UND FIBROBLASTEN WACHSTUMSFAKTOR ENTHALTENDE ZUSAMMENSETZUNGEN UND METHODEN ZUR INDUZIERUNG DER KARDIOGENESE
COMPOSITIONS A BASE DE PROTEINE MORPHOGENETIQUE OSSEUSE ET DE FACTEUR DE CROISSANCE DES FIBROBLASTES PERMETTANT D'INDUIRE UNE CARDIOGENESE ET METHODES AFFERENTES

(30) Priority: 16.08.1996 US 24062 P
(43) Date of publication of application: 09.09.1998
(73) Proprietor: MCW Research Foundation, Inc., Milwaukee Wisconsin 53226 (US)
(72) Inventor: LOUGH, John, W., Jr., Elm Grove, WI 53122-1731 (US); BARRON, Matthew, R., Wauwatosa, WI 53226 (US); BROGLEY, Michele, A., Milwaukee, WI 53219 (US); ZHU, Xiaolei, Milwaukee, WI 53213 (US); BAKER, John, E., Wauwatosa, WI 53213 (US)
(74) Representative: Fyles, Julie Marie
(86) International application number: PCT/US1997/014229
(87) International publication number: WO 1998/006420

(56) References cited:
- WO-A-93/05823
- US-A- 5 013 649
- CUMMINS, PETER ET AL: "Fibroblast and transforming growth factors in the heart: A role in cardiac growth?" DEV. CARDIOVASC. MED. ( 1993 ), 147(GROWTH FACTORS AND THE CARDIOVASCULAR SYSTEM), 17-30 , XP000946348
- KIMELMAN D ET AL: "SYNERGISTIC INDUCTION OF MESODERM BY FGF AND TGF-BETA AND THE IDENTIFICATION OF A MESSENGER RNA CODING FOR FGF IN THE EARLY XENOPUS EMBRYO" CELL, vol. 51, no. 5, 1987, pages 869-877, XP002148010 ISSN: 0092-8674
- FLORINI J R ET AL: "EFFECTS OF GROWTH FACTORS ON MYOGENIC DIFFERENTIATION" AMERICAN JOURNAL OF PHYSIOLOGY, vol. 256, no. 4 PART 1, 1989, pages C701-C711, XP000946376 ISSN: 0002-9513
- PARKER T G ET AL: "TGF -beta 1 and fibroblast growth factors selectively up-regulate tissue-specific fetal genes in cardiac muscle cells." CIBA FOUNDATION SYMPOSIUM, (1991) 157 152-60;DISCUSSION 161-4. , XP000946370
- EISENBERG C A ET AL: "ESTABLISHMENT OF THE MESODERMAL CELL LINE QCE-6. A MODEL SYSTEM FOR CARDIAC CELL DIFFERENTIATION" CIRCULATION RESEARCH,US,GRUNE AND STRATTON, BALTIMORE, vol. 78, no. 2, February 1996 (1996-02), pages 205-216, XP000865668 ISSN: 0009-7330
- NATURE, 07 January 1993, Vol. 361, NISWANDER et al., "FGF-4 and BMP-2 Have Opposite Effects on Limp Growth", pages 68-71.
- PROC. NATL. ACAD. SCI. U.S.A., January 1995, Vol. 92, MIMA et al., "Fibroblast Growth Factor Receptor is Required for in Vivo Cardiac Myocyte Proliferation at Early Embryonic Stages of Heart Development", pages 467-471.
- DEVELOPMENTAL BIOLOGY, 1995, Vol. 168, SUGI et al., "Activin-A and FGF-2 Mimic the Inductive Effects of Anterior Endoderm on Terminal Cardiac Myogenesis in Vitro", pages 567-574.
- DEVELOPMENTAL BIOLOGY, 1993, Vol. 157, SUGI et al., "Inhibition of Precardiac Mesoderm Cell Proliferation by Antisense Oligodeoxynucleotide Complementary to Fibroblast Growth Factor-2 (FGF-2)", pages 28-37.
- DEVELOPMENTAL BIOLOGY, 01 February 1997, Vol. 182, SASAI et al., "Ectodermal Patterning in Vertebrate Embryos", pages 5-20.
- DEVELOPMENTAL BIOLOGY, 25 August 1996, Vol. 178, LOUGH et al., "Combined BMP-2 and FGF-4, but Neither Factor Alone, Induces Cardiogenesis in Non-Precardiac Embryonic Mesoderm", pages 198-202.
- CELL, 25 July 1997, Vol. 90, NEUBUSER et al., "Antagonistic Interactions Between FGF and BMP Signalling Pathways: a Mechanism for Positioning the Sites of Tooth Formation", pages 247-255.

## Description

### Field of the Invention

The field of the present invention is a therapeutic composition that may be capable of inducing cardiogenesis in non-cardiac tissue. Specifically, the field of the present invention is a reagent comprising a transforming growth factor-β protein and a fibroblast growth factor protein.

### Background of the Invention

Dr. John Lough's laboratory has shown that the anterior lateral plate endoderm cells from the heart forming region (HFR) of stage 6 chicken embryos is specific in its ability to induce cardiogenesis in explanted precardiac mesoderm, a process highlighted by the formation of a multilayered vesicle of rhythmically contractile cells that express sarcomeric α-actin (Sugi, Y. and Lough, J., Dev. Dyn. 200: 155-162, 1994). Sugi and Lough recently reported that endoderm-associated factors, including members of the fibroblast growth factor (FGFs 1,2 and 4) and the transforming growth factor-beta (TGF-β) families (activin A), can mimic the cardiogenic effects of HFR endoderm on precardiac mesoderm (Sugi, Y. and Lough, J., Dev. Biol. 169:567-574, 1995). Several publications discuss mixture that include TGF-β and FGF-β in general. These include Eisenberg et al Circulation Research 78:205-216, 1996, Kimelman et al, Cell 5:869-877 , 1987, Cummins et al, Dev Cardiovasc Med, 147: 17-30, 1993. In addition WO 93/05823 in the name of Baylink discusses such general mixtures.

A further document in the name of Niswander et al, Nature: 361, 68-71, 1993 deals with mixtures of FGF-4 and BMP-2 which are expressed in the apical ectodermal ridge of the mouse limb to respectively stimulate and inhibit limb growth. However, in this document, when FGF-4 is used with BMP-2, the FGF-4 has an inhibiting effect on the BMP-2.

Finally, WO 96/15224 discloses a composition comprising a combination of growth factors for inducing the expression of tyrosine hydroxylase in neural cells, which may be used in the treatment of neurological disorders. However, this document does not mention using the composition to induce cardiogenesis.

The art now lacks a composition capable of inducing cardiogenesis in non-cardiac tissue using specified proteins.

### Disclosure of the Invention

The present invention relates to the use of a cardiogenic composition comprising a mixture of a purified bone morphogenetic protein 2 (BMP-2) and fibroblast growth factor -2 (FGF-2) in the manufacture of a medicament to induce cardiogenesis.

The composition of the present invention can be used in a method for inducing cardiogenesis in cells of a non-cardiac lineage comprising the steps of exposing cells to a mixture of purified bone morphogenetic protein-2 and a fibroblast growth factor (FGF-2) and observing the development of rhythmical contractile cells expressing sarcomeric α-actin. The exposure may be either in vitro or in vivo.

In one embodiment of the present invention, the protein mixture is applied exogenously to the cells in vitro. In another embodiment of the present invention, the cells are transformed with genetic constructs encoding bone morphogenetic protein and fibroblast growth factor. The genetic constructs are then allowed to express the cardiogenic proteins.

It is an important feature of the present invention that cardiac cells can be induced from non-cardiac tissue.

### Brief Description of the Drawings

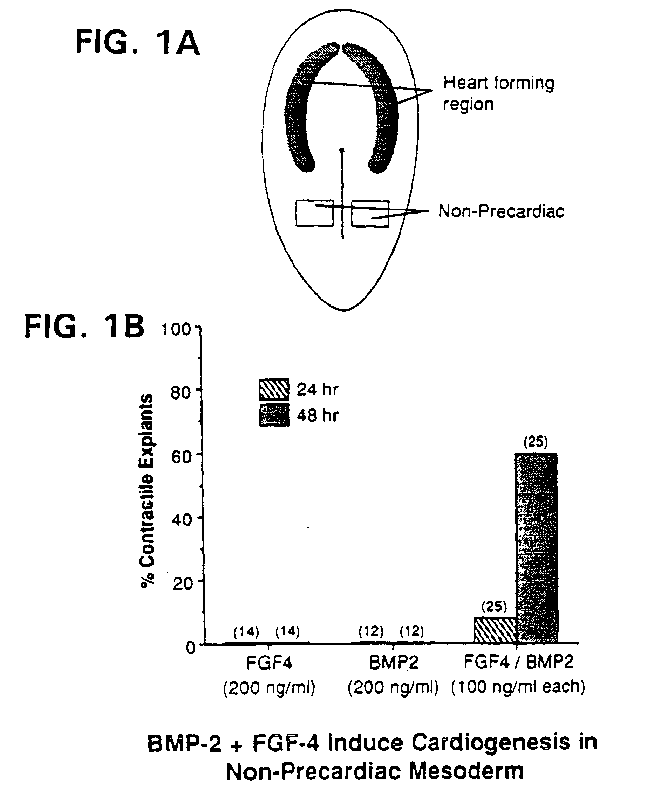

Fig. 1A and 1B describe the incidence of cardiogenesis in non-precardiac mesoderm cells treated with BMP-2 and FGF-4. Fig. 1A diagrams the heart-forming region (pre-cardiac tissue) and the non-precardiac tissue region of a stage 6 avian embryo. Fig. 1B is a graph of percent contractile explants that are obtained, versus treatment with FGF-4, BMP-2, or FGF-4 and BMP-2 combined.

### Best Modes for Carrying out the Invention

The present invention concerns the use of a cardiogenic composition comprising a mixture of a purified bone morphogenetic protein 2 (BMP-2) and fibroblast growth factor 2 (F6F-2) in the manufacture of a medicament for the induction of cardiogenesis in non-precardiac tissue, preferably human tissue. By "cardiogenesis" we mean the development of rhythmically and synchronously contractile cells that express sarcomeric α-actin from cells that are not part of the cardiac lineage. Preferably, a cell can be identified as a cardiac cell by visual observation via microscopy. The Examples below demonstrate that a monoclonal antibody that recognizes sarcomeric α-actin can confirm that cells are expressing α-actin.

By "non-precardiac tissue," we mean tissue that is not part of the cardiac lineage. For example, the following tissues are non-precardiac: fibroblasts, which make connective tissue, and cells that will become other mesoderm derivatives, such as skeletal muscle.

In one embodiment, the composition comprises a member of the bone morphogenetic protein subfamily of the transforming growth factor-*β* (TGF-*β*) family and a fibroblast growth factor (FGF). As an example, the reagent comprises a mixture of bone morphogenetic protein-2 (BMP-2) and FGF-4.

The composition comprises a "purified" mixture of proteins. By "purified" we mean that the proteins in question has been purified from native or recombinant bacterial sources. For example, a crude cell extract is "purified" as is a combination of proteins that have been individually purified to almost 100% homogeneity..

The experiments described below in the Example section demonstrate that combined BMP-2 and FGF-4, but neither factor alone, induce cardiogenesis in non-precardiac avian embryonic mesoderm. We envision that this combination of protein molecules, and perhaps other combinations of TGF-β and FGF family members, will induce cardiogenesis in other non-precardiac tissues, such as human non-cardiac tissue.

A typical source for BMP-2 is bone or recombinant human BMP-2 that is expressed in bacteria.

The compositions of the invention may comprise other therapeutically useful agents, including growth factors such as epidermal growth factor (EGF), transforming growth factor (TGF-β protein and an FGF protein, other therapeutically useful agents, including growth factors such as epidermal growth factor (EGF), transforming growth factor (TGF-α and TGF-β), activins, inhibins, and insulin-like growth factor (IGF).

The choice of matrix material is based on biocompatibility, biodegradability, mechanical properties, cosmetic appearance and interface properties. The particular application of the compositions will define the appropriate formulation. Potential matrices for the compositions may be biodegradable and chemically defined polymers, such as polymers of polylactic acid, polyglycolic polyorthoesters and polyanhydrides. Other potential materials are biodegradable and biologically well defined, such as collagen. Further matrices comprise pure proteins or extracellular matrix components. Other potential matrices are nonbiodegradable and chemically defined, such as sintered hydroxyapatite, bioglass, aluminates, or other ceramics. Matrices may comprise combinations of any of the above mentioned materials and other suitable types of material and may be altered in composition and processing to alter pore size, particle size, particle shape, and biodegradability.

Preferably, the bone morphogenetic protein and the fibroblast growth factor protein will be mixed in a 0.881 to 1.211 molar ratio. More preferably they will be mixed in a molar ratio. However, other ratios may result in cardiogenesis and may also be suitable.

The dosage regimen will be determined by the attending physician considering various factors which modify the action of the protein composition, e.g. amount of tissue desired to be formed, the site of tissue damage, the condition of the damaged tissue, the size of a wound, type of damaged tissue, the patient's age, sex, and diet, the severity of any infection, time of administration and other clinical factors. The dosage may vary with the type of matrix used in the reconstitution and the types of proteins in the composition. The addition of other known growth factors, such as IGF I (insulin like growth factor I), to the final composition, may also affect the dosage.

Potential uses of the compositions of the present compositions include use of the composition to treat patients with cardiac tissue damage or stress. The composition may be used as an adjunct to surgical procedures in which the composition may be applied directly to damaged or stressed tissue. In this embodiment, the composition may be used by itself or in conjunction with cultured cells which are capable of differentiation into cells of cardio- or cardiomyocyte lineage. Among the cells which may be useful in such an embodiment are cultured cardio- or cardiomyocytes, precursor cells to cardio- or cardiomyocyte lineage, fibroblasts, embryonic mesodermal cells, or earlier embryonic stem cells which are capable of differentiating into mesoderm.

Alternatively, the composition may be used to treat cells, whether autologous or heterologous, to promote the growth, proliferation, differentiation and/or maintenance of cells of a cardio- or cardiomyocyte lineage. The cells thus treated may then be applied to the damaged or stressed tissue, either alone or in conjunction with the protein composition of the present invention.

In another embodiment, DNA sequences encoding the proteins of the present compositions may be transfected into cells, rendering the cells capable of producing the BMP and FGF proteins. The transfected cells, which are capable of producing the BMP and FGF proteins, may then be implanted at the site of damaged or stressed tissue.

An appropriate matrix may be used with any of the above embodiments in order to maintain the composition and/or cells at the site of damaged or stressed tissue. Alternatively, an injectable formulation of the composition may be used for administration of the compositions of protein and/or cells. The above may also be used for prophylactic measure in order to prevent or reduce damage or stress to tissue.

### Examples

### 1. In General

Because immunostaining to detect additional TGF-β family growth factors in HFR endoderm revealed a provocative expression pattern for *Drosophila* decapentaplegic (dpp)-like proteins, we performed a degenerate reverse transcription/polymerase chain reaction (RT/PCR) screen to identify vertebrate dpp-like factors that are expressed by these cells. Among more than 50 PCR products sequenced to date, over half are identical to bone morphogenetic factor-2 (BMP-2).

We then investigated whether BMP-2 mimics the cardiogenic effects of HFR endoderm on precardiac mesoderm, as well as its ability to re-specify non-precardiac mesoderm to the cardiac lineage. We report here that, when present as the only supplement in defined medium, BMP-2 cannot support viability of either precardiac or non-precardiac mesoderm. Although FGF-4 can support cardiogenesis in precardiac mesoderm, this factor did not induce cardiogenesis in non-precardiac mesoderm, although explant growth was maintained. Remarkably, however, treatment of non-precardiac mesoderm with combined FGF-4 and BMP-2 induced cardiogenesis in a high incidence of explants, indicating that this combination of growth factors is able to re-specify embryonic cells to the cardiac lineage.

### 2. Materials and Methods

### Explantation and Culture of Embryonic Mesoderm:

Chicken embryos were staged according to the criteria of Hamburger and Hamilton (Hamburger, V. and H. L. Hamilton, J. Morphol. 38:49-92, 1951). Anterior lateral plate precardiac mesoderm, and non-precardiac mesoderm from the posterior half of stage 6 embryos, was micro-dissected, explanted to Lab-Tek chamber slides and cultured in M199 as previously described (Sugi, Y. and J. Lough, supra, 1994; Sugi, Y. and J. Lough, supra, 1995). Growth factors were added to the indicated final concentrations after explants attached to the fibrcnectin substrate. Human recombinant FGF-4 was purchased from R&D Systems. Human recombinant BMP-2 was provided by the Genetics Institute (Cambridge, MA). Medium, including growth factors, was changed daily.

### Immunohistochemistry:

Biochemical differentiation was monitored by immunohistochemistry, using a monoclonal antibody that recognizes sarcomeric α-actin (Sigma, Cat. No. A-2172); the secondary antibody was fluorescent isothiocyanate (FITC)-labeled goat anti-mouse IgM (Cappel). Decapentaplegic-like protein was localized using a polyclonal antibody (1:1,000) provided by Dr. F. Michael Hoffmann (University of Wisconsin; Panganiban, G.E.F., et al., Mol. Cell. Biol. 10:2669-2677, 1990) that recognizes Drosophila decapentaplegic. Controls consisted of identically diluted normal rabbit serum which was used as the primary antibody, and omission of the primary antibody. The secondary antibody was FITC-conjugated goat anti-rabbit IgG (1:500). All immunohistochemical procedures, including determinations of 5'-bromodeoxyuridine incorporation, have been previously described (Sugi, Y. and J. Lough, supra, 1995).

### Reverse Transcription-Polymerase Chain Reaction (RT/PCR) :

RNA from microdissected stage 6 HFR endoderm was purified, with 5 µg linear polyacrylamide as carrier, using RNAstat (Tel-Test, Inc.). Complementary DNA was synthesized by M-MLV reverse transcriptase-mediated extension of oligo-dT-primed RNA. To ensure the absence of contaminating genomic DNA, non-reverse transcribed RNA was simultaneously processed. Degenerate primers were designed to recognize conserved domains in the TGF-β dpp subfamily. The upstream primer was 5'-TGGAATTCGGITGGVAIGAYTGGAT-3' (96-fold degenerate) (SEQ ID NO:1); the reverse complement of the downstream target was 5'-GAGGATCCGGIACRCARCAIGCYTT-3' (128-fold degenerate) (SEQ ID NO : 2) .

Complementary DNAs were amplified using Thermus aquaticus (Taq) DNA polymerase (Promega) with 40 cycles of denaturation (94°C, 1 minute), primer annealing (45°C, 1.5 minutes) and extension (72°C, 2 minutes).
Complementary DNAs in the predicted 200 bp product were cloned into pCRScript (Stratagene). Identity of cloned inserts was determined by sequencing and comparison with the GenBank/EMBO database.

### 3. Results

### Immunohistochemical Localization of DPP-Like Protein in HFR Endoderm:

To ascertain whether *Drosophila* decapentaplegic (dpp)-like proteins were associated with HFR endoderm, the anti-dpp immunostaining pattern of cultured HFR endoderm was determined in comparison with explanted precardiac mesoderm. The periphery of HFR endoderm cells exhibited intense staining, which was not observed in precardiac mesoderm or in control explants stained with normal rabbit serum.

### RT/PCR Demonstration of BMP-2 in HFR Endoderm:

Because dpp is 75% homologous with BMPs 2 and 4, it was considered that the antigens described above represented these factors or perhaps other members of the TGF-β dpp subgroup. To identify dpp mRNAs that are expressed by HFR endoderm, a RT/PCR screen was performed using degenerate primers targeted to conserved domains in this subgroup. A single 200 bp PCR product, which was the predicted size for dpp cDNAs, was cloned and sequenced to identify individual dpp-like factors that are expressed by HFR endoderm. Among approximately 50 cDNAs sequenced to date, more than half were identical to BMP-2 over a 162 base stretch corresponding to nucleotides 800-962 of the chicken homologue (Francis, P.H., et al., Development 120:209-218, 1994), a domain specified by the primers. No other known or novel member of the dpp subgroup has been identified. Although the remaining, cloned cDNAs exhibited sequences that were similar to each other, these are not homologous to any database entries. These findings suggest that BMP-2 is the major member of the dpp group that is expressed by HFR endoderm.

### BMP-2 & FGF-4 Induce Cardiogenesis in Non-Precardiac Mesoderm (not part of the invention) :

Based on these results, it was of interest to determine whether BMP-2, when present alone in defined medium, could emulate the cardiogenic effect of HFR endoderm on precardiac mesoderm. Unlike FGFs (Sugi, Y. and J. Lough, supra, 1995; Zhu, X., et al., "Evidence for fibroblast growth factor 1 and 4 participation in paracrine and autocrine mechanisms that regulate embryonic heart development," submitted), BMP-2 supported neither survival nor differentiation of precardiac mesoderm. However, as anticipated, inclusion of FGF-4 with BMP-2 triggered terminal cardiogenesis in precardiac mesoderm.

Fig. 1A and B describe the incidence of cardiogenesis in non-precardiac mesoderm treated with BMP-2 and FGF-4. Non-precardiac mesoderm was explanted from the posterior half of stage 6 embryos and cultured in the presence of FGF-4 and/or 3MP-2 at the indicated concentrations. Whereas neither FGF-4 nor BMP-2 alone induced cardiogenic differentiation in any explant, the majority of explants treated with both growth factors exhibited cellular multilayering and rhythmic contractility within 1 or 2 days. Referring to Fig. 1B, numbers in parentheses indicate experimental repetitions that were conducted during the aggregate of five experiments, each of which included approximately five replicate explants; the incidence of differentiation (40-60%) was similar within each experimental repetition.

As diagramed in Fig. 1A, these determinations were performed on non-precardiac mesoderm explanted from the posterior region of stage 6 embryos. Cells in this area are destined to become extraembryonic mesoderm and lateral plate mesoderm that is not cardiogenic (review: Nicolet, G., Adv. Morphogenesis 9:231-262, 1971). As shown in Fig. 1B, neither FGF-4 nor BMP-2 alone could induce formation of contractile explants in non-precardiac mesoderm. Cells cultured with BMP-2 alone detached from the culture dish and did not survive; and, although treatment with FGF-4 alone supported cellular proliferation as evidenced by 5'-bromodeoxyuridine incorporation, differentiation was never observed. Remarkably however, the combined presence of FGF-4 and BMP-2 caused cardiogenic differentiation in over half of the non-orecardiac mesoderm explants (Fig. 1B), as indicated by formation of a multicellular vesicle which exhibited rhythmic contractility and sarcomeric α-actin differentiation. Because differentiation of non-precardiac mesoderm was not usually observed until the second day in vitro (Fig. 1B), in contrast to precardiac mesoderm in which differentiation is observed on day one, the occurrence of a re-specification step in non-precardiac mesoderm explants is suggested. These findings indicate that these growth factors synergistically function to induce cardiogenesis in cells that are not fated to the cardiac lineage.

### 4. Discussion

In addition to verifying our previous findings that HFR endoderm is specific in its ability to induce terminal differentiation (Sugi, Y. and J. Lough, supra, 1994), Schultheiss, et al. (Schultheiss, T.M., et al., Development 121:4203-4214, 1995) also reported that HFR endoderm is capable of re-specifying embryonic cells to the cardiogenic lineage. Regarding the latter, our observation that HFR endoderm was unable to re-specify posterior non-precardiac mesoderm (Sugi, Y. and J. Lough, supra, 1994) apparently reflected inadequate local concentrations of endodermal factors, caused by placing the explants at opposite sides of the culture dish rather than in contiguity. The findings reported herein chat the combined activities of BMP-2 and FGF-4, both of which are expressed in HFR endoderm, induce cardiogenesis in non-precardiac mesoderm are consistent with the ability of HFR endoderm to re-specify cells to the cardiogenic lineage.

We previously determined that HFR endoderm expresses three members of the FGF family (FGFs 1, 2 and 4), each of which supports differentiation of precardiac mesoderm (Sugi, Y. and J. Lough, supra, 1995; Zhu, X., et al., supra, in press, Developmental Dynamics). Moreover, experiments utilizing sodium chlorate to specifically prevent binding of FGF to cognate receptors have indicated that FGF signaling is obligatory for endoderm-mediated cardiogenesis (unpublished observations). However, because the major role of FGF is apparently to regulate precardiac mesoderm proliferation, cooperation with "differentiation" factors such as BMP-2 may be required to induce cardiac myocyte differentiation. In this interpretation, our observations that isolated precardiac mesoderm can differentiate in culture with FGF alone suggests that these cells had been signaled by BMP-2 prior to explantation from the embryo. Although cooperativity between activin and FGFs has been shown to regulate mesoderm induction (Cornell, R. and D. Kimelman, Development 120:453-462, 1994; LaBonne C. and M. Whitman, Develonment 120:463-472, 1994) in Xenopus, our preliminary findings indicate that activin cannot replace BMP-2 in supporting the differentiation of non-precardiac mesoderm. And, although FGF-4 and BMP-2 are expressed in the apical ectodermal ridge of the mouse limb bud to respectively stimulate and inhibit limb growth (Niswander L. and G.R. Martin, Nature 361:68-71, 1993), BMP-2's inhibitory effect on growth could be considered as a consequence of its differentiative function. Finally, the possibility that the BMPs have differentiative potency has recently been reported by Pourquie, et al. (1995) who demonstrated that BMP-4 specifies myotome cells to the skeletal myocyte lineage (Pourquie, O., et al., Cell 84:461-472, 1995).

### 5. Prophetic Results Of Animal Experiments

It is anticipated that ongoing experiments using a rabbit model will reveal that the combination of bone morphogenetic protein (BMP) and fibroblast growth factor (FGF), as opposed to either factor alone, will significantly augment protection of the heart (cardioprotection) from an experimentally-induced transient episode of ischemia and reperfusion.

Rationale: The rationale for these experiments is as follows. Basic fibroblast growth factor (bFGF; FGF-2) alone has been shown to increase functional recovery in isolated rats heart when given immediately prior to induction of ischemia and reperfusion (Padua, R.R., et al., Mol. Cell Biochem. 143:129-135, 1995). This cardioprotective effect was achieved using a dose of 10 *µ*g FGF-2 per rat heart, administered directly into the coronary vasculature. In dogs, moreover, FGF-2 treatment has recently been shown to reduce the size of myocardial infarctions, 7 days after reperfusion without hemodynamic effects or evidence of neovascularization; this cardioprotective effect was achieved when FGF-2 was given intracoronary at a dose of 10 *µ*g per dog heart prior to reperfusion (Horrigan, M.C.G., et al., Circulation 94:1927-1933, 1996).

Osteogenic protein-1 (hOP-1) is a member of the TGF-β superfamily that is closely related to BMP. hOP-1 given intravenously prior to reperfusion at a dose of 20 µg/animal reduced reperfusion injury 24 hours later in an in vivo rat model of coronary artery ischemia and reperfusion (Lefer, A.M., et al., J. Mol. Cell Cardiol. 24:585-593, 1992).

FGF-4 + BMP-2: We recently discovered that combined FGF-4, or FGF-2, plus BMP-2 induces embryonic mesoderm cells that are not destined to the cardiogenic lineage to differentiate into a multilayered, synchronously contractile hollow vesicle that resembles a microscopic heart in vitro; treatment with FGF-4 (or FGF-2) alone, or BMP-2 alone, had not effect (Lough, J., et al., Developmental Biology 178:198-202, 1996). From this result we predict that BMP functions in the embryo to recruit cells to the cardiogenic lineage, and that FGF is required to support these cells along the cardiac differentiative pathway. Unlike the situation in the embryo, cells in the adult heart neither grow nor develop; as a result, damage suffered during insults such as myocardial infarction is frequently catastrophic. The reason(s) adult cardiac myocytes are unable to undergo growth and regeneration is unknown. It is our expectation that the unavailability of growth factors such as FGF and BMP in a functionally useful context is a major cause for the inability of cells in the adult heart to regenerate.

### Experimental Plan: General

Regarding cardioprotection, we are unaware of any studies examining the role of BMP *per se*, either alone or in conjunction with other growth factors, in maintaining cardiac function or protecting the myocardium from the effect of ischemia and reperfusion, a situation that is encountered in patients undergoing elective ishemia, such as heat bypass surgery and angioplasty, and heart attacks. It is our expectation that, by combining BMP-2 and FGF-2, the cardioprotective effect described above for FGF-2 alone will be augmented. Accordingly, we are determining the physiological advantage of treatment with combined growth factors on pre-ischemic aerobic cardiac function and post-ischemic recovery following ischemia, using a protocol which is routinely used in the laboratory of one of the inventors, Dr. John Baker. The experimental model utilizes the isolated rabbit heart, which is subjected to one cycle of ischemia and reperfusion. Recovery of heart function from the ischemia/reperfusion insult will be used as an endpoint to determine the effectiveness of the combined growth factor treatment. The basis of this cardioprotective effect will be determined at the cellular and molecular level in terms of whether renewed cardiac myocyte growth and differentiation occur, using techniques that are routine in Dr. Lough's laboratory.

### Experimental Plan: Specific

Experiments will be performed in a random manner using four groups to determine whether BMP-2 synergistically improves the cardioprotective effect of FGF-2. The four experimental groups are as follows:
Group 1, saline vehicle control (no growth factors) - I.V. route of administration
Group 2, BMP-2 (80 *µ*g/kg i.v.) alone
Group 3, FGF-2 (40 *µ*g/kg i.v.) alone
Group 4, BMP-2 (80 *µ*g/kg i.v.) in combination with FGF-2 (40 *µ*g/kg i.v.)

Dosages and intended routes of administration are based on published reports that have demonstrated cardioprotective effects of FGF and BMP-related growth factors (1-3). Each group will consist of eight (8) immature rabbits, 7-10 days old; by this neonatal stage of development, the heart is fully functional, consisting of cardiac myocytes which have recently ceased their normal proliferation. For all groups, treatments will be initiated 24 hours prior to heart excision and study to allow time for these agents to exert a (presumptively) prophylactic effect on the heart. Twenty-four hours after treatment the aorta will be cannulated and perfusion of the isolated heart will be immediately commenced with bicarbonate buffer at 39°C in the Langendorff mode at a constant pressure. Saline-filled latex balloons will be placed in the left and right ventricles for measurement of developed pressures. Hearts will be perfused for a 30 minute equilibration period during which time biventricular function and corcnary flow rate will be recorded under steady-state conditions. Hearts will be subjected to a 30 minute period of global, no-flow ischemia at 39°C. After the ischemic period, heart function will again be measured under steady-state conditions. Thus, each heart serves as its own control. Recovery of cardiac function will be expressed as a percentage of its pre-ischemic value. The results will be evaluated by comparing pre-ischemic and post-ischemic function within the growth factor-treated groups (Groups 2-4) with recovery in the control group (Group 1).

### Industrial Applicability

The present invention is a therapeutic composition capable of inducing cardiogenesis in non-cardiac tissue. The composition is a purified mixture of transforming growth factor β-protein and fibroblast growth factor.

### SEQUENCE LISTING

(1) GENERAL INFORMATION:
   (i) APPLICANT:
      (A) NAME: MCW Research Foundation, Inc.
      (B) STREET: 8701 Watertown Plank Road
      (C) CITY: Milwaukee
      (D) STATE: Wisconsin
      (E) COUNTRY: United States of America
      (F) POSTAL CODE: 53226
      (G) TELEPHONE: (414) 456-4402
      (H) TELEFAX: (414) 266-8522
   (ii) TITLE OF INVENTION: CARDIOGENESIS COMPOSITION
   (iii) NUMBER OF SEQUENCES: 2
   (iv) CORRESPONDENCE ADDRESS:
      (A) ADDRESSEE: Quarles & Brady
      (B) STREET: 411 East Wisconsin Avenue
      (C) CITY: Milwaukee
      (D) STATE: Wisconsin
      (E) COUNTRY: U.S.A.
      (F) ZIP: 53202-4497
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Baker, Jean C.
      (B) REGISTRATION NUMBER: 35,433
      (C) REFERENCE/DOCKET NUMBER: 650053.91134
   (ix) TELECOMMUNICATION INFORMATION:
      (A) TELEPHONE: (414) 277-5709
      (B) TELEFAX: (414) 271-3552
(2) INFORMATION FOR SEQ ID NO:1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: Oligonucleotide
   (ix) FEATURE:
      (A) NAME/KEY: misc_difference
      (B) LOCATION: replace (11..12, " ")
      (D) OTHER INFORMATION: /note= "Position 11 is listed as N but was inosine."
   (ix) FEATURE:
      (A) NAME/KEY: misc_difference
      (B) LOCATION: replace(17..18, "")
      (D) OTHER INFORMATION: /note= "Position 17 is listed as N but was inosine."
   (xi) SEQUENCE DESCRIPTION: SEQ ID NO:1:
   TGGAATTCGG NTGGVANGAY TGGAT                                    25
(2) INFORMATION FOR SEQ ID NO:2:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: Oligonucleotide
   (ix) FEATURE:
      (A) NAME/KEY: misc_difference
      (B) LOCATION: replace(11..12, "")
      (D) OTHER INFORMATION: /note= "Position 11 is listed as N but was inosine."
   (ix) FEATURE:
      (A) NAME/KEY: misc_difference
      (B) LOCATION: replace(20..21, "")
      (D) OTHER INFORMATION: /note= "Position 20 is listed as N but was inosine."
   (xi) SEQUENCE DESCRIPTION: SEQ ID NO:2:
   GAGGATCCGG NACRCARCAN GCYTT                                    25

## Claims

1. Use of a cardiogenic composition comprising a mixture of a purified bone morphogenetic protein 2 (BMP-2) and fibroblast growth factor 2 (FGF-2) in the manufacture of a medicament to induce cardiogenesis.

2. Use according to Claim 1 wherein the ratio of BMP-2 and FGF-2 is a 1:1 molar ratio.

3. Use according to Claim 1 or Claim 2 wherein the composition additionally comprises a matrix material.

4. Use according to Claim 3 wherein the matrix material is collagen.

## Patentansprüche

1. Verwendung einer kardiogenen Zusammensetzung, die ein Gemisch aus gereinigtem morphogenetischen Knochenprotein 2 (BMP-2) und Fibroblastenwachstumsfaktor 2 (FGF-2) umfasst, zur Herstellung einer Medikaments zur Induktion einer Kardiogenese.

2. Verwendung nach Anspruch 1, wobei das Verhältnis von BMP-2 und FGF-2 ein Molverhältnis von 1:1 ist.

3. Verwendung nach Anspruch 1 oder 2, wobei die Zusammensetzung zusätzlich ein Matrixmaterial umfasst.

4. Verwendung nach Anspruch 3, wobei das Matrixmaterial Kollagen ist.

## Revendications

1. Utilisation d'une composition cardiogènique comprenant un mélange d'une protéine 2 morphogénétique osseuse (BMP-2) et d'un facteur de croissance 2 de fibroblaste (FGF-2) dans la fabrication d'un médicament pour induire une cardiogénèse.

2. Utilisation selon la revendication 1, dans laquelle le rapport molaire de la BMP-2 sur le FGF-2 est de 1:1.

3. Utilisation selon la revendication 1 ou 2, dans laquelle la composition comprend en outre un matériau formant matrice.

4. Utilisation selon la revendication 3, dans laquelle le matériau formant matrice est le collagène.
